(19) Europäisches Patentamt
European Patent Office
Office européen des brevets

(11) EP 0 630 229 B1

(12) **EUROPÄISCHE PATENTSCHRIFT**

(45) Veröffentlichungstag und Bekanntmachung des Hinweises auf die Patenterteilung: **23.07.1997 Patentblatt 1997/30**

(21) Anmeldenummer: **93906483.8**

(22) Anmeldetag: **04.03.1993**

(51) Int. Cl.$^6$: **A61K 9/00**

(86) Internationale Anmeldenummer: **PCT/EP93/00496**

(87) Internationale Veröffentlichungsnummer: **WO 93/18746 (30.09.1993 Gazette 1993/24)**

(54) **DRUCKGASPACKUNGEN UNTER VERWENDUNG VON POLYOXYETHYLEN-GLYCERYL-OLEATEN**

PRESSURIZED GAS PACKAGINGS USING POLYOXYETHYLENE GLYCERYL-OBATES

CONDITIONNEMENT SOUS GAZ COMPRIME UTILISANT DES OLEATES DE POLYONYETHYLENE ET DE GLYCERYLE

(84) Benannte Vertragsstaaten:
**AT BE CH DE DK ES FR GB GR IE IT LI LU MC NL PT SE**

(30) Priorität: **17.03.1992 DE 4208505**
**08.05.1992 DE 4215188**
**16.09.1992 DE 4230876**

(43) Veröffentlichungstag der Anmeldung: **28.12.1994 Patentblatt 1994/52**

(73) Patentinhaber: **ASTA Medica Aktiengesellschaft**
**D-01277 Dresden (DE)**

(72) Erfinder:
- **HETTCHE, Helmut**
  **D-6057 Dietzenbach (DE)**
- **ENGEL, Jürgen**
  **D-8755 Alzenau (DE)**
- **MUCKENSCHNABEL, Reinhard**
  **D-6000 Frankfurt (Main) 60 (DE)**

(56) Entgegenhaltungen:
**EP-A- 0 203 211**
**WO-A-87/05210**
**WO-A-87/05211**

- **JOURNAL DE PHARMACIE DE BELGIQUE Bd. 28, Nr. 2, 1973, BRUXELLES (BE) Seiten 209 - 216 I. DONTCHEVA, ET AL. 'etude comparee de l'effet stabilisant des**
- **substances tensio-actives sur des systemes disperses heterogenes'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 630 229 B1

**Beschreibung**

Aerosol-Druckgaspackungen werden seit vielen Jahren für die unterschiedlichsten Zwecke verwendet. Unter Aerosol-Druckgaspackungen versteht man druckfeste Behältnissse, aus denen eine unter Druck stehende Michung aus verflüssigtem Treibgas und Wirkstoff durch Betätigung eines Ventils freigesetzt wird. Druckgaspackungen sind beispielsweise in Sucker, Fuchs und Speiser (Herausgeber), Pharmazeutische Technologie, Thieme, Stuttgart, 1991, S. 673 - 688 beschrieben, weiter werden Aerosole und Druckgaspackungen in List, Arzneiformenlehre, Wissenschaftliche Verlagsgesellschaft, Stuttgart, 1985, S. 8 - 18 und in Voigt, Lehrbuch der pharmazeutischen Technologie, VCh, Weinheim, 1987 auf den Seiten 427 - 436 beschrieben. Weiter wird diese populäre Darreichungsform von Thoma, Aerosole, Selbstverlag, Frankfurt am Main, 1979 ausführlich erläutert. Im medizinischen Bereich werden sie vorteilhaft dann eingesetzt, wenn Wirkstoffe direkt in die Lunge transportiert und dort deponiert werden sollen. Der Vorteil der Aerosol-Druckgaspackungen besteht darin, daß bei ihrer Verwendung eine Wolke feinstzerteilter Partikel entsteht, die vom Patienten eingeatmet werden kann. In der Folge kommt es zu einem raschen Wirkungseintritt am Wirkort Lunge, was für die Therapie beispielsweise des Asthma bronchiale von entscheidender Wichtigkeit ist. Auf der anderen Seite kann bei der Prävention von Asthmaanfällen durch prophylaktisch wirksame Substanzen bei derartig lokaler Applikation direkt in die Lunge die Dosierung niedrig gehalten werden. Damit ist das Auftreten von unerwünschten Nebenwirkungen minimiert im Vergleich zur Applikation über den Magen/Darm-Trakt.

Aerosol-Druckgaspackungen haben daher eine weite Verbreitung in der Therapie von Atemwegserkrankungen gefunden. Sie sind einfach, sicher und preiswert. Mögliche Probleme bei der Koordination von Atemzug des Patienten und Auslösung eines Aerosolstoßes können entweder durch zwischen Aerosolpackung und Mund des Patienten eingeschobene Expansionskammern ("Spacer") oder durch spezielle Konstruktionen der Inhalatoren vermieden werden, bei denen der Einatemzug des Patienten den Aerosolstoß auslöst.
Neben der inhalativen Anwendung zur Prophylaxe von Asthma bronchiale und zur Therapie des akuten Asthmaanfalls kann die erfindungsgemäße Formulierung auch als Nasenspray und zur Anwendung als Mundspray (linguale und bukkale Applikation) gelangen.

Als Treibmittel für Dosieraerosole setzte man bisher FCKW ein (fluorierte chlorierte Kohlenwasserstoffe). Verwendbar als Treibmittel sind zum Beispiel folgende fluorierte chlorierte Kohlenwasserstoffe und Kohlenwasserstoffe: Pentan, n-Butan, iso-Butan, TG 11, TG 12, TG 21, TG 22, TG 23, TG 113, TG 114, TG 115, TG 142b und TG C 318.
Die Typenbezeichnung der fluorierten Chlorkohlenwaserstoffe leitet sich aus folgendem Schlüsselsystem ab:

| | |
|---|---|
| Zahl in Einerstelle | = Anzahl Fluoratome (F) |
| Zahl in Zehnerstelle minus 1 | = Anzahl Wasserstoffatome (H) |
| Zahl in Hunderterstelle plus 1 | = Anzahl Kohlenstoffatome (C) |
| Zahl der noch freien Valenzen | = Anzahl Chloratome (Cl) |

Seit Aufstellung der Ozontheorie (Abbau des stratosphärischen Ozons durch FCKW und andere chlorhaltige organische Verbindungen) sucht man als Treibmittel geeignete Flüssiggase, die weder brennbar, noch in der Lage sind, Ozon abzubauen und außerdem nicht gesundheitsschädlich sind.

Seit einiger Zeit arbeitet man mit nichtchlorierten Fluorkohlenwasserstoffen wie beispielsweise 1,1,1,2-Tetrafluorethan (TG 134a) oder 2H-Heptafluorpropan (TG 227).

Außer TG 134a und TG 227 wäre noch TG 152a (Difluorethan, $CH_3CHF_2$), TG 143a (Trifluorethan, $CH_3CF_3$) und TG 161 (Fluorethan, $CH_3CH_2F$) zu nennen.

Nachteilig an diesen Treibmitteln ist allerdings, daß zu ihrer Verwendung erforderliche Suspensionsstabilisatoren und Ventilschmiermittel nicht in ausreichendem Maße in ihnen löslich sind. So erfordert die Verwendung von TG 134a etwa 25% Ethanol, um das bisher in Aerosolsuspensionen verwendete Sorbitantrioleat (Span®85) in ausreichendem Maße zu lösen (s. EP-A-O 372 777). Beispielsweise können gemäß EP-A-O 372 777 auch folgende Verbindungen verwendet werden: ein- und mehrwertige Alkohole wie zum Beispiel Isopropanol oder Glycerin, Kohlenwasserstoffe wie zum Beispiel Hexan, Heptan oder Pentan und Ether wie Dimethylether.

Nachteilig bei einer derartig hohen Konzentration ist, daß es zu Auflösungserscheinungen für den in der Suspension befindlichen Wirkstoff kommen kann und damit die Gefahr des Teilchenwachstums besteht. Wachsen beim Lagern einer derartigen Suspension die Wirkstoffteilchen über eine Größe von 10 µm, so kann es einerseits zu Verstopfungen des Aerosolventils, auf der anderen Seite aber zu einer Wirkungsverringerung bis zu Unwirksamkeit des Aerosols kommen, da die Wirkstoffpartikel aufgrund ihrer Größe nicht mehr in der Lage sind, tiefere Lungenabschnitte zu erreichen.

In I. Dontcheva et al., J. Pharm. Belg., 1973, 28, 2, 209 - 216 wird die stabilisierende Wirkung von verschiedenen nichtionogenen oberflächenaktiven Stoffen, u. a. von verschiedenen Tagaten, auf Suspensionen von Phenoxymethylpenicillin beschrieben, bei gleichzeitiger Betrachtung des HLB-Wertes. Hierbei wird die Verwendung der Tagate jedoch ausschließlich in wäßrigen Suspensionen und Emulsionen des Phenoxymethylpenicillins dargestellt.

Ebenso wird in EP-A-O 203 211 die Verwendung von Tagaten in einer wäßrigen Emulsion zur dermalen Anwendung mitgeteilt.

Nach H. P. Fiedler, Lexikon der Hilfsstoffe für Pharmazie, Kosmetik und angrenzende Gebiete, 3. Aufl., Editio Cantor Aulendorf, Seite 1 203 sind Tagate im wesentlichen Lösungsvermittler, etwa für etherische Öle, Riechstoffe, Vitaminöle, Wirkstoffe. Es werden die HLB-Werte der Tagattypen aufgeführt und festgestellt, daß Tagate gut haut- und schleimhautverträglich sind.

Aus WO 87/05210 ist die Verwendung von Glycerin-Polyethylenglykoloxyloleat, einer Esterverbindung der Hydroxyölsäure oder Ricinolsäure bekannt. Beschrieben wird die Verwendung dieser Verbindung als Lösungsvermittler für Benzodiazepine in halogenierten Kohlenwasserstoffen.

In WO 87/05211 wird die Verwendung von Glycerin-Polyethylenglykoloxyoleat als Lösungsvermittler für Nifedipin in Treibmitteln wie halogenierten Fluorkohlenwasserstoffen beschrieben, wobei der Wirkstoff insbesondere gelöst vorliegt und sublingual appliziert wird.

Es besteht also ein dringender Bedarf nach Substanzen, die

- physiologisch verträglich sind
- technologisch geeignet sind, Aerosolsuspensionen von TG 134a oder TG 227 zu stabilisieren sowie die Funktion des Dosierventils zu verbessern
- in TG 134a oder TG 227 ohne oder unter Anwendung geringster Mengen anderer physiologisch verträglicher Lösungsvermittler löslich sind
- geschmacklich akzeptabel sind.

Es wurde nun überraschend gefunden, daß Polyoxyethylen-25-glyceryl-trioleat eine Substanz mit den eben erwähnten erforderlichen Eigenschaften darstellt (Handelsname "Tagat® TO"; Hersteller: Goldschmidt, Essen) Polyoxyethylen-25-glyceryl-trioleat hat folgende Strukturformel:

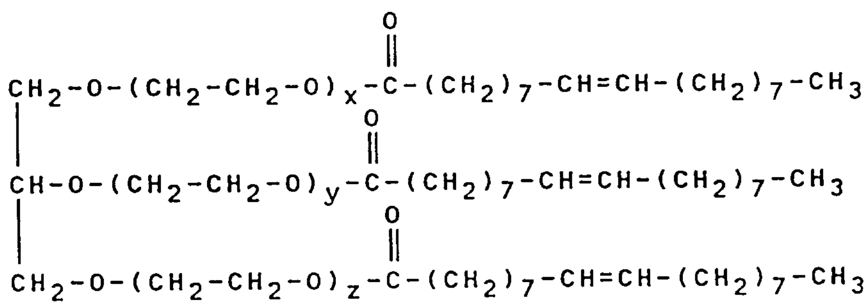

Formel I

wobei gilt: $x+y+z \simeq 20 - 30$

Der HLB-Wert beträgt 11,3; die Hydroxylzahl liegt zwischen 18 und 33; die Säurezahl erreicht höchstens 2; die Verseifungszahl liegt zwischen 75 und 90 und die Jodzahl zwischen 34 und 40.

Die Hydroxylzahl wurde nach DGF-C-V 17a, die Säurezahl nach DGF-C-V 2, die Verseifungszahl nach DGF-C-V 3 und die Jodzahl nach DGF-C-V 11 bestimmt. Polyoxylethylen-25-glyceryl-trioleat ist eine bernsteinfarbige Flüssigkeit.

Polyoxyethylen-glyceryl-trioleat ist

- physiologisch verträglich (vergleichbar mit Sorbitantrioleat (= Span® 85))
- technologisch geeignet, Aerosolsuspension von TG 134a und/oder TG 227 zu stabilisieren sowie die Funktion des Dosierventils zu verbessern.
- in TG 134a bzw. TG 227 löslich unter gleichzeitiger Anwesenheit von weniger als 1-2 % Ethanol oder vergleichbarer Alkohole
- geschmacklich akzeptabel

Weitere, als Suspensionsstabilisatoren geeignete Verbindungen, die die vorstehend erwähnten Eigenschaften aufweisen sind:

- Tagat® O

  Tagat® O ist chemisch ein Polyoxyethylen-30-glyceryl-monooleat.

  Polyoxyethylen-30-glyceryl-monooleat hat folgende Strukturformel:

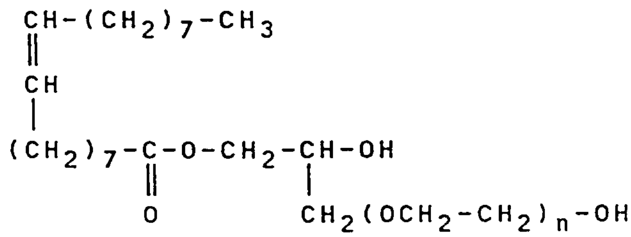

Formel II

wobei n: ca. 30.

Der HLB-Wert beträgt 16.4; die Hydroxylzahl liegt zwischen 50 und 65; die Säurezahl erreicht höchstens 2; die Verseifungszahl liegt zwischen 30 und 45 und die Jodzahl zwischen 15 und 19.

- Tagat® 02

  Tagat® 02 ist chemisch ein Polyoxyethylen-20-glyceryl-monooleat.

  Polyoxyethylen-20-glyceryl-monooleat hat folgende Strukturformel

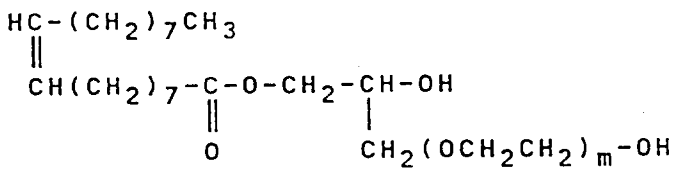

Formel III

m: ca. 20.

Der HLB-Wert beträgt 15.0; die Hydroxylzahl liegt zwischen 70 und 85; die Säurezahl erreicht höchstens 2; die Verseifungszahl liegt zwischen 40 und 55 und die Jodzahl zwischen 21 und 27.

Das Lösungsvermögen einer Mischung von TG 134a oder TG 227 mit 1-2 % Ethanol für die üblichen Wirksubstanzen ist derartig gering, daß es keine Rolle für ein mögliches Kristallwachstum der Wirkstoffe spielt. Die bisher in Handelspräparaten eingesetzten Suspensionsstabilisatoren hatten einen HLB-Wert von unter 5 (z.B. Span 85: HLB = 1.8) und liegen damit im Bereich der W/O-Emulgatoren. (Voigt, Lehrbuch der pharmazeutischen Technologie, Weinheim, 1987, S. 332) Es ist daher überraschend, daß eine Substanz wie Polyoxyethylen-25-glyceryl-trioleat, Polyoxyethylen-30-glyceryl-monooleat und Polyoxyethylen-20-glyceryl-monooleat mit einem HLB-Wert von 11.3 bis 16.4 für diesen Zweck geeignet ist.

Die eingesetzte Menge an Polyoxyethylen-25-glyceryl-trioleat, Polyoxyethylen-30-glyceryl-monooleat und Polyoxyehtylen-20-glyceryl-monooleat bezogen auf eine eingesetzte Menge Wirkstoff beträgt beispielsweise zwischen 0,3 und 8000, insbesondere 5 und 4000 und besonders bevorzugt zwischen 15 und 2500 Gewichts %.

Als Wirkstoffe können eingesetzt werden:

Analgetika, Antiallergika, Antibiotika, Anticholinergika, Antihistaminika, antiinflammatorisch wirkende Substanzen, Antitussiva, Bronchodilatatoren, Diuretika, Enzyme, Herz-Kreislauf wirksame Substanzen, Hormone, Proteine und Peptide. Beispiele für Analgetika sind Codein, Diamorphin, Dihydromorphin, Ergotamin, Fentanyl, Morphin; Beispiele für Antiallergika sind Cromo-glicinsäure, Nedocromil; Beispiele für Antibiotika sind Cephalosporine, Fusafungin, Neomycin, Penicilline, Pentamidin, Streptomycin, Sulfonamide, Tetracycline; Beispiele für Anticholinergika sind Atropin, Atropinmethonitrat, Ipratropiumbromid, Oxitropiumbromid, Trospiumchlorid; Beispiele für Antihistaminika sind Azelastin, Flezelastin, Methapyrilen; Beispiele für antiinflammatorisch wirksame Substanzen sind Beclomethason, Budesonid, Dexamethason, Flunisolid, Fluticason, Tipredane, Triamcinolon; Beispiele für Antitussiva sind Narcotin, Noscapin; Beispiele für Bronchodilatatoren sind Bambuterol, Bitolterol, Carbuterol, Clenbuterol, Ephedrin, Epinephrin, Formoterol, Fenoterol, Hexoprenalin, Ibuterol, Isoprenalin, Isoproterenol, Metaproterenol, Orciprenalin, Phenylephrin, Phenylpropanolamin, Pirbuterol, Procaterol, Reproterol, Rimiterol, Salbutamol, Salmeterol, Sulfonterol, Terbutalin, Tolobuterol; Beispiele für Diuretika sind Amilorid, Furosemid; ein Beispiel für Enzyme ist Trypsin; Beispiele für Herz-Kreislauf wirksame Substanzen sind Diltiazem und Nitroglycerin; Beispiele für Hormone sind Cortison, Hydrocortison, Prednisolon; Beispiele für Proteine und Peptide sind Cyclosporine, Cetrorelix, Glucagon, Insulin. Weitere Wirkstoffe, die eingesetzt werden können, sind Adrenochrom, Colchicin, Heparin, Scopolamin.

Es können auch Kombinationen der vorstehend aufgeführten Substanzen eingesetzt werden.

Die beispielhaft angeführten Wirkstoffe können als freie Basen oder Säuren oder als pharmazeutisch verträgliche

Salze eingesetzt werden. Als Gegenionen können beispielsweise physiologisch verträgliche Erdalkali- oder Alkalimetalle oer Amine sowie beispielsweise Azetat, Benzolsulfonat, Benzoat, Hydrogencarbonat, Hydrogentartrat, Bromid, Chlorid, Iodid, Karbonat, Citrat, Fumarat, Malat, Maleat, Gluconat, Lactat, Pamonat, Hydroxynaphtoat und Sulphat eingesetzt werden. Es können auch Ester eingesetzt werden, zum Beispiel Acetat, Acetonid, Propionat, Dipropionat, Valerat.
Die Menge an Tagat(R)TO, Tagat(R)O oder Tagat(R)02, bezogen auf die Gesamtmischung, zusammengesetzt aus Wirkstoffen, Treibgas oder Teibgasgemischen, gegebenenfalls Hilfsstoffen, beträgt beispielsweise 0,01 Gew.% - 5 Gew.%, insbesondere 0,2 Gew.% - 2,5 Gew.% und besonders bevorzugt 0,75 Gew.% - 1,5 Gew.%.

Der Zusatz von Cosolventien ist möglich,
beispielsweise aliphatische Alkohole mit 2 bis 6 C-Atomen oder deren Ester oder Ketone oder Polygole. Beispiele sind Ethanol, Isopropanol, Proylenglykol, Aceton, Essigsäurethylester, n-Propanol, vorzugsweise Ethanol und Isopropanol.

Die Menge an Ethanol oder Isopropanol, bezogen auf die Gesamtmischung, beträgt zwischen 0 Gew.% bis 10 Gew,%, Insbesondere 0,1 Gew.% bis 2 Gew.% und besonders bevorzugt 0,2 Gew.% bis 1 Gew.%.

Der Zusatz weiterer grenzflächenaktiver Substanzen, wie beispielsweise in EP 0 372 777 aufgeführt, ist selbstverständlich möglich.

Die Suspendierung der Wirkstoffe kann entweder erfolgen bei normalem Luftdruck, wobei das Suspensionsmedium auf niedrige Temperaturen abgekühlt werden muß (zum Beispiel - 35°C bis - 55°C) oder innerhalb eines Druckgefäßes, wobei bei Normaltemperaturen (Raumtemperatur 15° bis 25°C) gearbeitet werden kann.
Die Suspension wird homogenisiert und anschließend in Druckdosen abgefüllt, die mit einem Dosierventil verschlossen sind oder anschließend verschlossen werden.

Beispiel 1

1000 g 2H-Heptafluorpropan (= Treibmittel 227) werden auf eine Temperatur von etwa -55°C abgekühlt und unter Rühren mit einer Lösung aus aus 11,7 g Polyoxyethylen-25-glyceryl-trioleat (Handelsname: Tagat® TO, Goldschmidt AG) in 11,7 g absolutem Ethanol versetzt. Anschließend werden 16,8 g mikronisierte Cromoglicinsäure, Dinatriumsalz und 8,4 g mikronisiertes Reproterolhydrochlorid sowie 0,9 g mikronisiertes Saccharin-Natrium und 6,75 g Pfefferminzöl zugesetzt und die entstandene Suspension intensiv homogenisiert. Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 50 µl der Suspension freisetzen. Pro Hub werden damit 1 mg Cromoglicinsäure, Dinatriumsalz und 0,5 mg Reproterolhydrochlorid freigesetzt.

Beispiel 2

1000 g 2H-Heptafluorpropan (= Treibmittel 227) werden auf eine Temperatur von etwa -55°C abgekühlt und unter Rühren mit einer Lösung aus aus 11,7 g Polyoxyethylen-30-glyceryl-monooleat (Handelsname: Tagat® O, Goldschmidt AG) in 11,7 g absolutem Ethanol versetzt. Anschließend werden 16,8 g mikronisierte Cromoglicinsäure, Dinatriumsalz und 8,4 g mikronisiertes Reproterolhydrochlorid sowie 0,9 g mikronisiertes Saccharin-Natrium und 6,75 g Pfefferminzöl zugesetzt und die entstandene Suspension intensiv homogenisiert. Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 50 µl der Suspension freisetzen. Pro Hub werden damit 1 mg Cromoglicinsäure, Dinatriumsalz und 0,5 mg Reproterolhydrochlorid freigesetzt.

Beispiel 3

1000 g 2H-Heptafluorpropan (= Treibmittel 227) werden auf eine Temperatur von etwa -55°C abgekühlt und unter Rühren mit einer Lösung aus aus 11,7 g Polyoxyethylen-20-glyceryl-monooleat (Handelsname: Tagat® O2, Goldschmidt AG) in 11,7 g absolutem Ethanol versetzt. Anschließend werden 16,8 g mikronisierte Cromoglicinsäure, Dinatriumsalz und 8,4 g mikronisiertes Reproterolhydrochlorid sowie 0,9 g mikronisiertes Saccharin-Natrium und 6,75 g Pfefferminzöl zugesetzt und die entstandene Suspension intensiv homogenisiert. Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 50 µl der Suspension freisetzen. Pro Hub werden damit 1 mg Cromoglicinsäure, Dinatriumsalz und 0,5 mg Reproterolhydrochlorid freigesetzt

Beispiel 4

Es wird gearbeitet wie in Beispiel 1, jedoch wird statt 16,8 g mikronisierter Cromoglicinsäure, Dinatriumsalz und 8,4 g mikronisiertem Reproterolhydrochlorid 16,8 g mikronisiertes D-18024 eingesetzt.
Pro Hub werden damit 1 mg D-18024 freigesetzt.

D-18024 hat folgende Strukturformel:

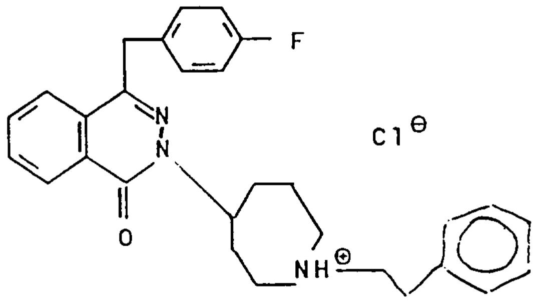

D-18024 trägt den INN Flezelastinhydrochlorid.

Beispiel 5

Es wird gearbeitet wie in Beispiel 1, jedoch wird statt 16,8 g mikronisiertem Cromoglicinsäure, Dinatriumsalz, 8,4 g mikronisiertem Reproterolhydrochlorid, 0,9 g mikronisiertem Saccharin-Natrium und 6,75 g Pefferminzöl 4,2 g mikronisiertes Budesonid eingesetzt.
Ein Hub enthält 0,25 mg Budesonid.

Beispiel 6

1000 g Heptafluorpropan (=Treibmittel 227) werden auf eine Temperatur von etwa -55° C abgekühlt und unter Rühren mit einer Mischung aus 11,7 g Polyoxyethylen-25-glyceryl-trioleat (Handelsname: Tagat(R) TO, Goldschmidt AG) und 6,75 g Dentomint PH 799 959 (Hersteller: Haarmann und Reimer, Holzminden) versetzt. Unter weiterem Rühren und Kühlen werden 16,8 g mikronisierte Cromoglicinsäure, Dinatriumsalz und 8,4 g mikronisiertes Reproterolhydrochlorid sowie 0,9 g mikronisiertes Saccharin-Natrium zugesetzt und die entstehende Suspension intensiv homogenisiert.
Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 50 µl der Suspension freisetzen. Pro Hub werden damit 1 mg Cromoglicinsäure, Dinatriumsalz und 0,5 mg Reproterolhydrochlorid freigesetzt.

**Patentansprüche**

1. Aerosol-Druckgaspackungen zur Verabreichung von medizinisch aktiven Substanzen, dadurch gekennzeichnet, daß Polyoxyethylen-25-glyceryl-trioleat gemäß Formel I

$$\begin{array}{l} \qquad\qquad\qquad\quad O \\ \qquad\qquad\qquad\quad \| \\ CH_2\text{-}O\text{-}(CH_2\text{-}CH_2\text{-}O)_x\text{-}C\text{-}(CH_2)_7\text{-}CH{=}CH\text{-}(CH_2)_7\text{-}CH_3 \\ | \qquad\qquad\qquad\quad O \\ | \qquad\qquad\qquad\quad \| \\ CH\text{-}O\text{-}(CH_2\text{-}CH_2\text{-}O)_y\text{-}C\text{-}(CH_2)_7\text{-}CH{=}CH\text{-}(CH_2)_7\text{-}CH_3 \\ | \qquad\qquad\qquad\quad O \\ | \qquad\qquad\qquad\quad \| \\ CH_2\text{-}O\text{-}(CH_2\text{-}CH_2\text{-}O)_z\text{-}C\text{-}(CH_2)_7\text{-}CH{=}CH\text{-}(CH_2)_7\text{-}CH_3 \end{array}$$

worin für $x + y + z \cong 20 - 30$ gilt,
als Stabilisator für nichtwäßrige Suspensionen und/oder als Ventilschmiermittel verwendet wird.

2. Aerosol-Druckgaspackungen zur Verabreichung von medizinisch aktiven Substanzen, dadurch gekennzeichnet, daß Polyoxyethylen-30-glyceryl-monooleat gemäß Formel II

$$\begin{array}{lll} CH\text{-}(CH_2)_7\text{-}CH_3 & & \\ \| & & \\ CH & & \\ | & & \\ (CH_2)_7\text{-}C\text{-}O\text{-}CH_2\text{-}CH\text{-}OH & & \\ \qquad\quad \| \qquad\qquad | & & \\ \qquad\quad O \qquad\quad CH_2(OCH_2\text{-}CH_2)_n\text{-}OH & & \end{array}$$

wobei für n etwa 30 gilt,
als Stabilisator für nichtwäßrige Suspensionen und/oder als Ventilschmiermittel verwendet wird.

3. Aerosol-Druckgaspackungen zur Verabreichung von medizinisch aktiven Substanzen, dadurch gekennzeichnet, daß Polyoxyethylen-20-glyceryl-monooleat gemäß Formel III

$$\begin{array}{l} HC\text{-}(CH_2)_7\text{-}CH_3 \\ \;\| \\ CH(CH_2)_7\text{-}C\text{-}O\text{-}CH_2\text{-}CH\text{-}OH \\ \qquad\qquad\quad \| \qquad\qquad | \\ \qquad\qquad\quad O \qquad\quad CH_2(OCH_2CH_2)_m\text{-}OH \end{array}$$

wobei für m etwa 20 gilt,
als Stabilisator für nichtwäßrige Suspensionen und/oder als Ventilschmiermittel verwendet wird.

4. Aerosol-Druckgaspackungen gemäß Anspruch 1, dadurch gekennzeichnet, daß der Polyoxyethylen-25-glyceryl-trioleat-Anteil gemäß Formel I

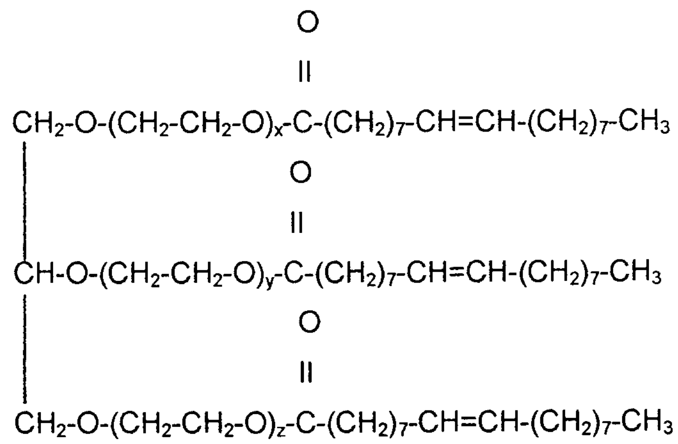

bezogen auf das Gesamtgewicht der Mischung zwischen 0,01 und 5 Gewichts% liegt.

**5.** Aerosol-Druckgaspackungen gemäß Anspruch 1, dadurch gekennzeichnet, daß der Polyoxyethylen-25-glyceryl-trioleat-Anteil gemäß Formel I

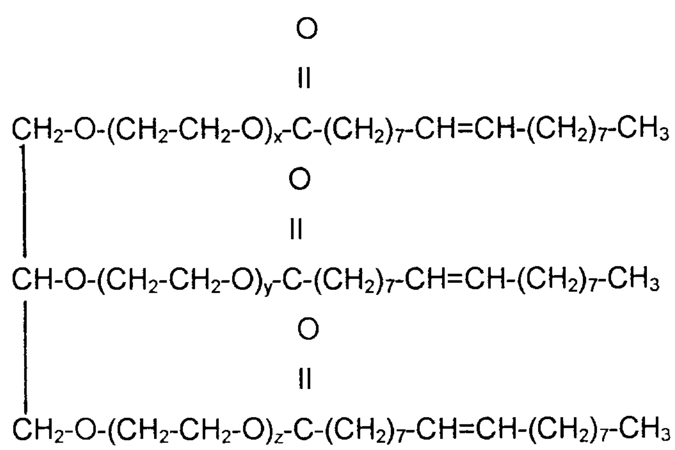

bezogen auf das Gesamtgewicht der Mischung, zwischen 0,2 und 2,5 Gewichts% liegt.

**6.** Aerosol-Druckgaspackungen gemäß Anspruch 1, dadurch gekennzeichnet, daß der Polyoxyethylen-25-glyceryl-trioleat-Anteil, gemäß Formel I

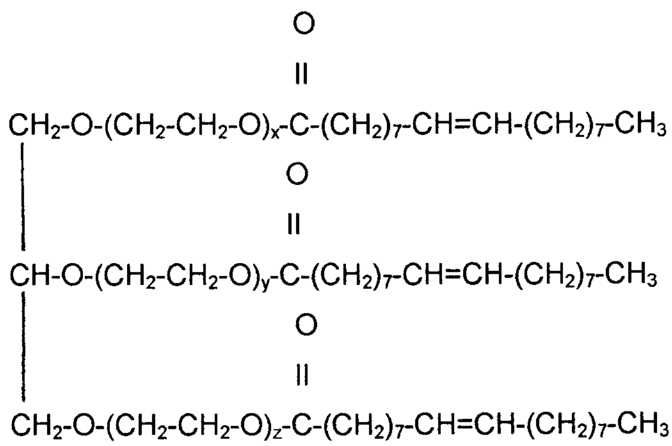

bezogen auf das Gesamtgewicht der Mischung, zwischen 0,75 und 1,5 Gewichts% beträgt.

7. Aerosol-Druckgaspackungen gemäß Anspruch 2, dadurch gekennzeichnet, daß der Polyoxyethylen-30-glycerylmonooleat-Anteil gemäß Formel II

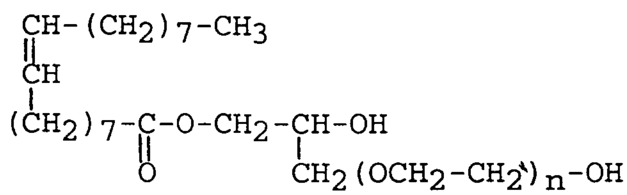

bezogen auf das Gesamtgewicht der Mischung zwischen 0,01 und 5 Gewichts% liegt.

8. Aerosol-Druckgaspackungen gemäß Anspruch 2, dadurch gekennzeichnet, daß der Polyoxyethylen-30-glycerylmonooleat-Anteil gemäß Formel II

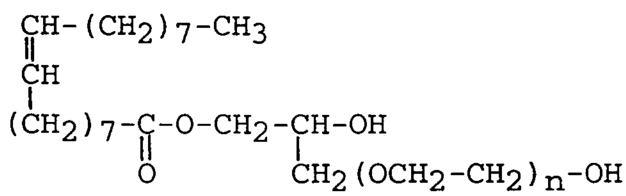

bezogen auf das Gesamtgewicht der Mischung zwischen 0,2 und 2,5 Gewichts% liegt.

9. Aerosol-Druckgaspackungen gemäß Anspruch 2, dadurch gekennzeichnet, daß der Polyoxyethylen-30-glycerylmonooleat-Anteil gemäß Formel II

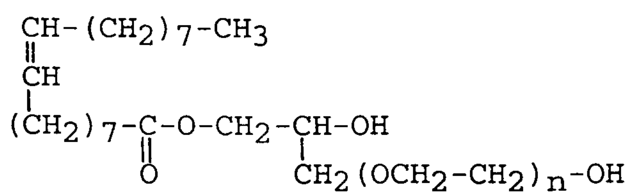

bezogen auf das Gesamtgewicht der Mischung zwischen 0,75 und 1,5 Gewichts% liegt.

10. Aerosol-Druckgaspackungen gemäß Anspruch 3, dadurch gekennzeichnet, daß der Polyoxyethylen-20-glyceryl-monooleat-anteil gemäß Formel III

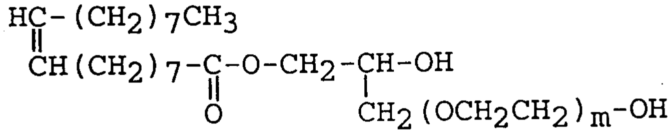

bezogen auf das Gesamtgewicht der Mischung, zwischen 0,01 und 5 Gewichts% liegt.

11. Aerosol-Druckgaspackungen gemäß Anspruch 3, dadurch gekennzeichnet, daß der Polyoxyethylen-20-glyceryl-monooleat-Anteil gemäß Formel III

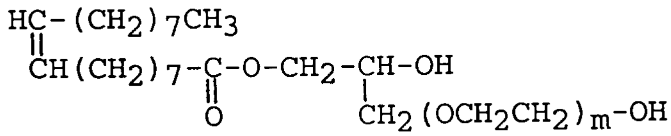

bezogen auf das Gesamtgewicht der Mischung, zwischen 0,2 und 2,5 Gewichts% liegt.

12. Aerosol-Druckgaspackungen gemäß Anspruch 3, dadurch gekennzeichnet, daß der Polyoxyethylen-20-glyceryl-monooleat-Anteil gemäß Formel III

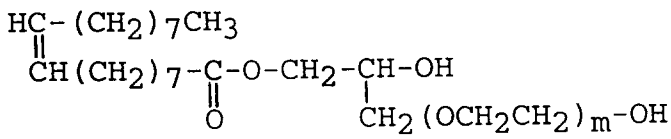

bezogen auf das Gesamtgewicht der Mischung, zwischen 0,75 und 1,5 Gewichts% liegt.

13. Aerosol-Druckgaspackungen gemäß Anspruch 1 bis 12 dadurch gekennzeichnet, daß als Treibmittel TG 227 und/oder TG 134a verwendet wird.

14. Verfahren zur Herstellung von Aerosol-Druckgaspackungen gemäß Anspruch 1 bis 12, dadurch gekennzeichnet, daß das Treibmittel TG 227 und/oder TG 134a unter Kühlung oder unter Druck mit Polyoxyethylen-25-glyceryl-trioleat und/oder Polyoxyethylen-30-glyceryl-monooleat und/oder Polyoxyethylen-20-glyceryl-monooleat sowie ggf. mit Cosolventien, danach mit Wirkstoffen und ggf. Hilfsstoffen versetzt und die so erhaltene Suspension homogenisiert und mit Treibmittel TG 227 und/oder TG 134a aufgefüllt und in druckfeste Behältnisse abgefüllt wird.

## Claims

1. Aerosol compressed gas packages for the administration of medically active substances, characterised in that polyoxyethylene 25 glyceryl trioleate of the formula I

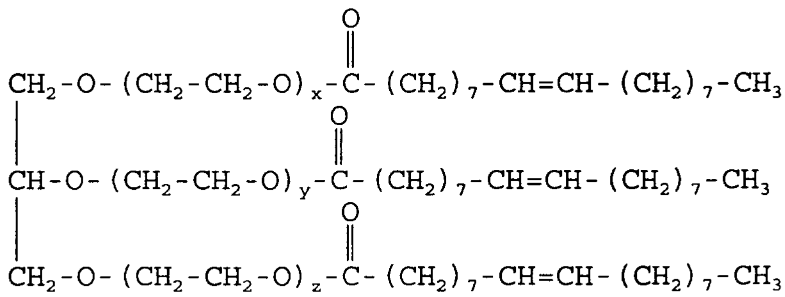

in which x + y + z ≅ 20 - 30 applies, as a stabiliser for non-aqueous suspensions and/or as a valve lubricant.

**2.** Aerosol compressed gas packages for the administration of medically active substances, characterised in that polyoxyethylene 30 glyceryl monooleate of the formula II

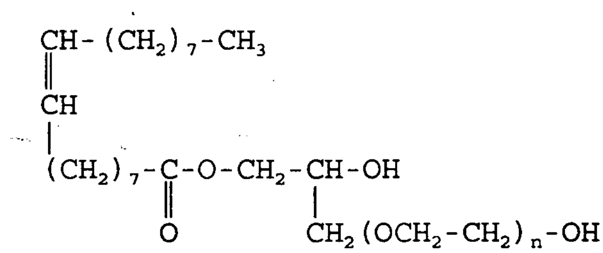

wherein n is approximately 30, as a stabiliser for non-aqueous suspensions and/or as a valve lubricant.

**3.** Aerosol compressed gas packages for the administration of medically active substances, characterised in that polyoxyethylene 20 glyceryl monooleate of the formula III

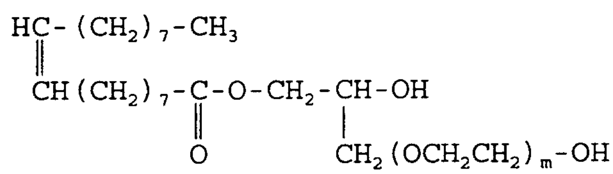

wherein m is approximately 20, as a stabiliser for non-aqueous suspensions and/or as a valve lubricant.

**4.** Aerosol compressed gas packages according to claim 1, characterised in that the content of polyoxyethylene 25 glyceryl trioleate of the formula I

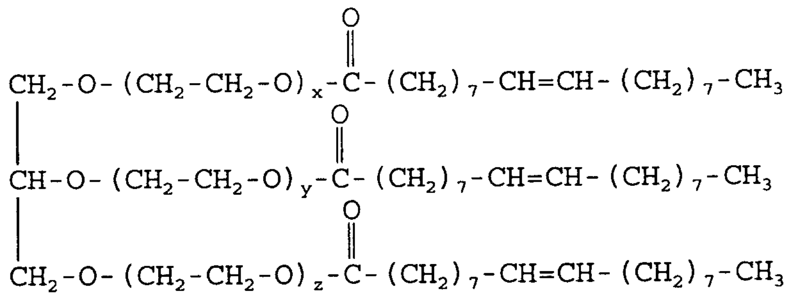

is between 0.01 and 5 wt.% relative to the total weight of the mixture.

5. Aerosol compressed gas packages according to claim 1, characterised in that the content of polyoxyethylene 25 glyceryl trioleate of the formula I

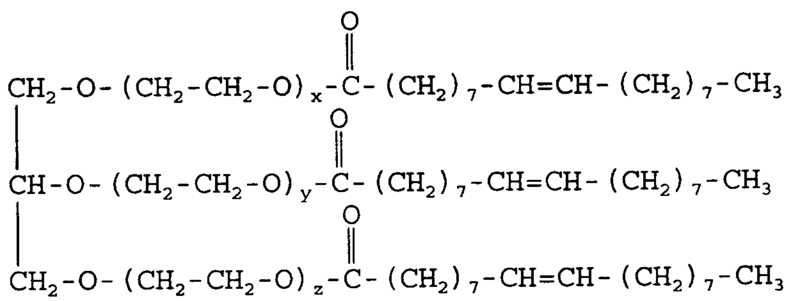

is between 0.2 and 2.5 wt.% relative to the total weight of the mixture.

6. Aerosol compressed gas packages according to claim 1, characterised in that the content of polyoxyethylene 25 glyceryl trioleate of the formula I

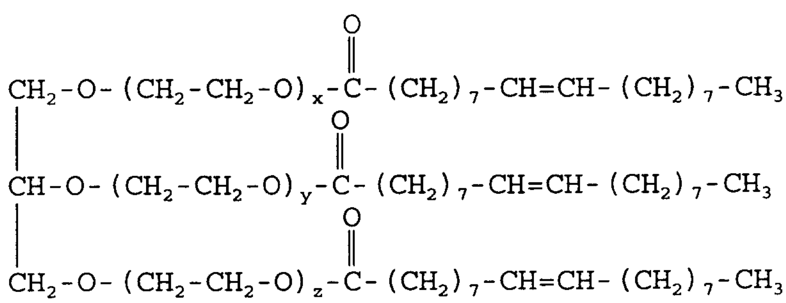

is between 0.75 and 1.5 wt.% relative to the total weight of the mixture.

7. Aerosol compressed gas packages according to claim 2, characterised in that the content of polyoxyethylene 30 glyceryl monooleate of the formula II

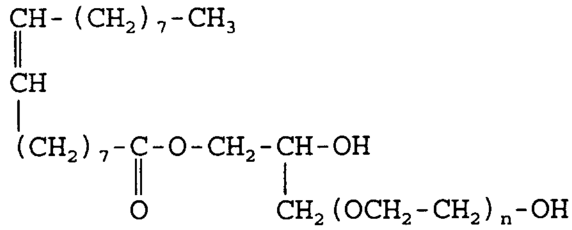

is between 0.01 and 5 wt.% relative to the total weight of the mixture.

**8.** Aerosol compressed gas packages according to claim 2, characterised in that the content of polyoxyethylene 30 glyceryl monooleate of the formula II

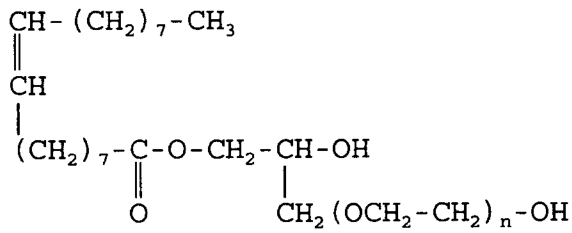

is between 0.2 and 2.5 wt.% relative to the total weight of the mixture.

**9.** Aerosol compressed gas packages according to claim 2, characterised in that the content of polyoxyethylene 30 glyceryl monooleate of the formula II

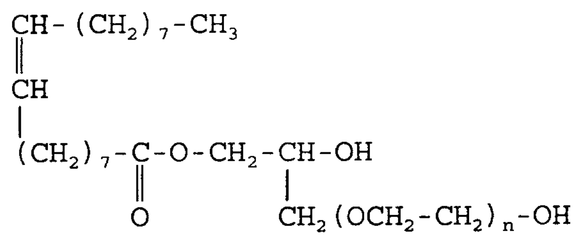

is between 0.75 and 1.5 wt.% relative to the total weight of the mixture.

**10.** Aerosol compressed gas packages according to claim 3, characterised in that the content of polyoxyethylene 20 glyceryl monooleate of the formula III

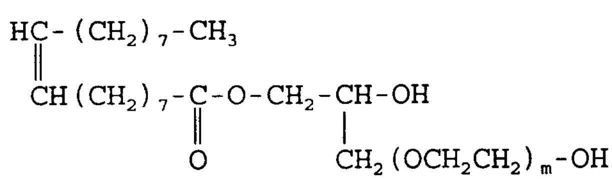

is between 0.01 and 5 wt.% relative to the total weight of the mixture.

**11.** Aerosol compressed gas packages according to claim 3, characterised in that the content of polyoxyethylene 20 glyceryl monooleate of the formula III

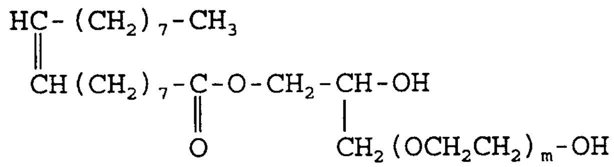

is between 0.2 and 2.5 wt.% relative to the total weight of the mixture.

12. Aerosol compressed gas packages according to claim 3, characterised in that the content of polyoxyethylene 20 glyceryl monooleate of the formula III

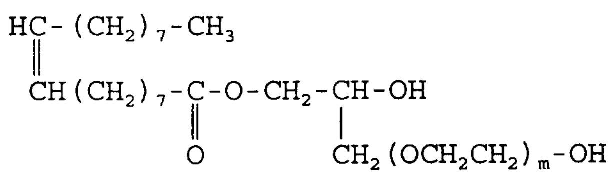

is between 0.75 and 1.5 wt.% relative to the total weight of the mixture.

13. Aerosol compressed gas packages according to claims 1 to 12, characterised in that TG 227 and/or TG 134a are used as the propellant.

14. Process for the production of aerosol compressed gas packages according to claims 1 to 12, characterised in that the propellant TG 227 and/or TG 134a is combined with cooling or under pressure with polyoxyethylene 25 glyceryl trioleate and/or polyoxyethylene 30 glyceryl monooleate and/or polyoxyethylene 20 glyceryl monooleate and optionally with cosolvents, is then combined with active substances and optionally auxiliary substances and the resultant suspension is homogenised and made up with propellant TG 227 and/or TG 134a and filled into pressure-resistant containers.

**Revendications**

1. Conditionnements sous gaz comprimé pour aérosol en vue de l'administration de substances médicalement actives,
caractérisés en ce qu'
on utilise le trioléate de polyoxyéthylène-25-glycéryle selon la formule I :

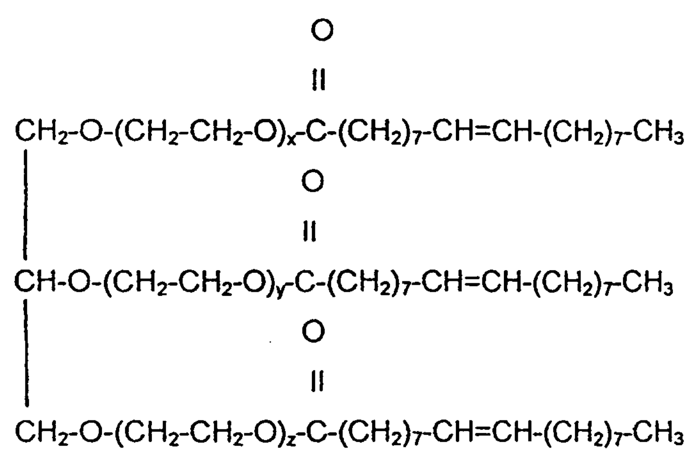

dans laquelle, les valeurs $x + y + z \cong 20 - 30$ sont valables,

comme agent stabilisant pour suspension non aqueuses et/ou comme agent lubrifiant pour soupapes.

**2.** Conditionnements sous gaz comprimé pour aérosol en vue de l'administration de substances médicalement actives,
caractérisés en ce qu'
on utilise le monooléate de polyoxyéthylène-30-glycéryle selon la formule II :

$CH\text{-}(CH_2)_7\text{-}CH_3$
||
CH
|
$(CH_2)_7\text{-}C\text{-}O\text{-}CH_2\text{-}CH\text{-}OH$
||          |
O          $CH_2(OCH_2\text{-}CH_2)_n\text{-}OH$

dans laquelle n : environ 30 s'applique pour n,
en tant qu'agent stabilisant pour suspension non aqueuses et/ou agent lubrifiant pour soupapes.

**3.** Conditionnements sous gaz comprimé pour l'administration de substances médicalement actives,
caractérisés en ce qu'
on utilise le monooléate de polyoxyéthylène-20-glycéryle selon la formule III :

$HC\text{-}(CH_2)_7\text{-}CH_3$
||
$CH(CH_2)_7\text{-}C\text{-}O\text{-}CH_2\text{-}CH\text{-}OH$
||          |
O          $CH_2(OCH_2CH_2)_m\text{-}OH$

dans laquelle pour m environ 20 s'applique
en tant qu'agent stabilisant pour suspension non aqueuses et/ou agent lubrifiant pour soupapes.

**4.** Conditionnements sous gaz comprimé pour aérosol selon la revendication 1,
caractérisés en ce que
la proportion de trioléate de polyoxyéthylène-25-glycéryle selon la formule I :

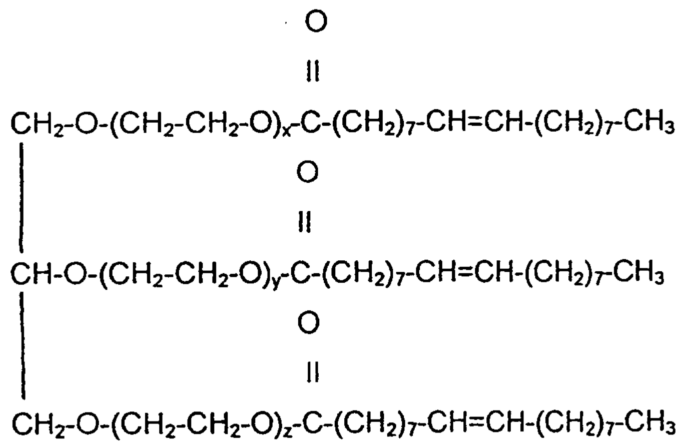

se situe, rapporté au poids total de mélange, entre 0,01 et 5 % en poids.

5. Conditionnements sous gaz comprimé pour aérosol selon la revendication 1, caractérisés en ce que la proportion de trioléate de polyoxyéthylène-25-glycéryle selon la formule I :

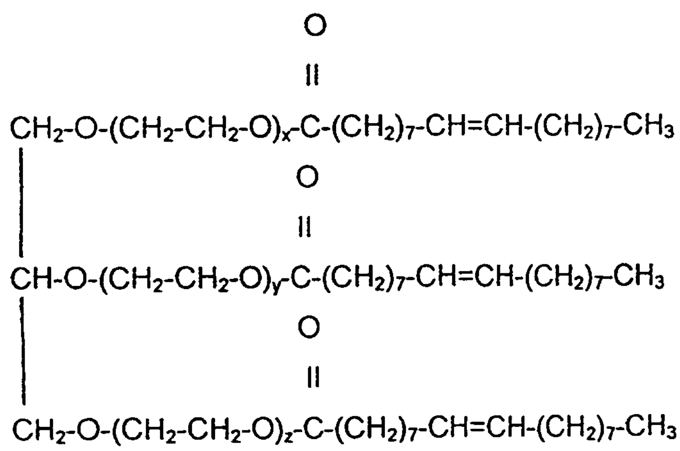

se situe, rapporté au poids total de mélange, entre 0,2 et 2,5 % en poids.

6. Conditionnements sous gaz comprimé pour aérosol selon la revendication 1, caractérisés en ce que la proportion de trioléate de polyoxyéthylène-25-glycéryle selon la formule I :

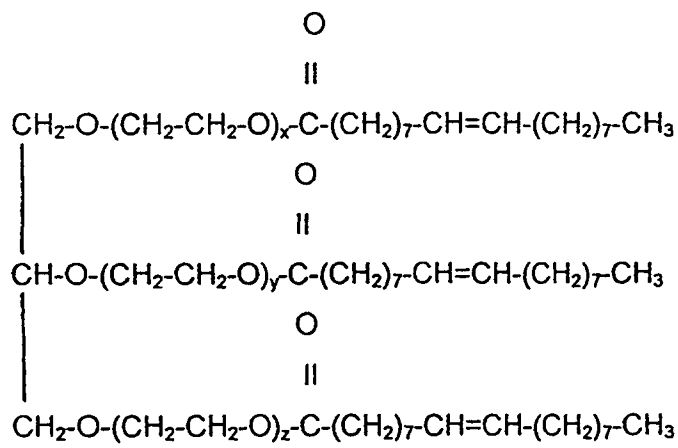

s'élève à, rapporté au poids total de mélange, entre 0,75 et 1,5 % en poids.

7. Conditionnements sous gaz comprimé pour aérosol selon la revendication 2, caractérisés en ce que la proportion de monooléate de polyoxyéthylène-30-glycéryle selon la formule II :

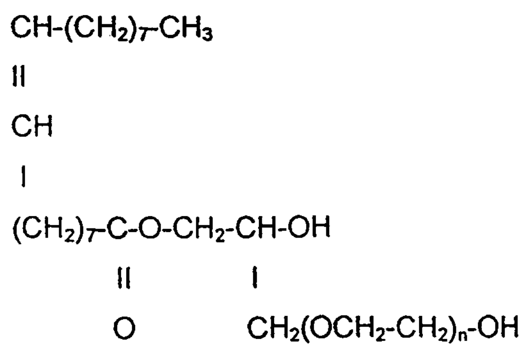

se situe, rapporté au poids total de mélange, entre 0,01 et 5 % en poids.

8. Conditionnements sous gaz comprimé pour aérosol selon la revendication 2, caractérisés en ce que la proportion de monooléate de polyoxyéthylène-30-glycéryle selon la formule II :

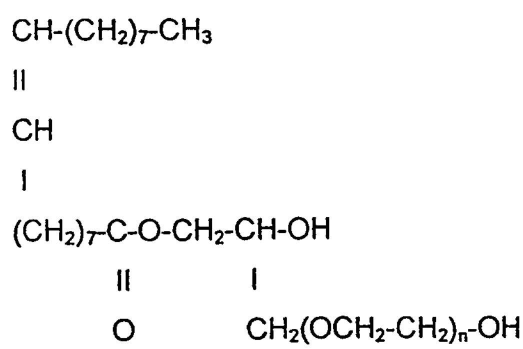

se situe, rapporté au poids total de mélange, entre 0,2 et 2,5 % en poids.

**9.** Conditionnements sous gaz comprimé pour aérosol selon la revendication 2, caractérisés en ce que la proportion de monooléate de polyoxyéthylène-30-glycéryle selon la formule II :

$CH\text{-}(CH_2)_7\text{-}CH_3$

II

CH

I

$(CH_2)_7\text{-}C\text{-}O\text{-}CH_2\text{-}CH\text{-}OH$

II I

O $CH_2(OCH_2\text{-}CH_2)_n\text{-}OH$

se situe, rapporté au poids total de mélange, entre 0,75 et 1,5 % en poids.

**10.** Conditionnements sous gaz comprimé pour aérosol selon la revendication 3, caractérisés en ce que la proportion de monooléate de polyoxyéthylène-20-glycéryle selon la formule III :

$HC\text{-}(CH_2)_7\text{-}CH_3$

II

$CH(CH_2)_7\text{-}C\text{-}O\text{-}CH_2\text{-}CH\text{-}OH$

II I

O $CH_2(OCH_2CH_2)_m\text{-}OH$

se situe, rapporté au poids total de mélange, entre 0,01 et 5 % en poids.

**11.** Conditionnements sous gaz comprimé pour aérosol selon la revendication 3, caractérisés en ce que la proportion de monooléate de polyoxyéthylène-20-glycéryle selon la formule III :

$HC\text{-}(CH_2)_7\text{-}CH_3$

II

$CH(CH_2)_7\text{-}C\text{-}O\text{-}CH_2\text{-}CH\text{-}OH$

II I

O $CH_2(OCH_2CH_2)_m\text{-}OH$

se situe, rapporté au poids total de mélange, entre 0,2 et 2,5 % en poids.

**12.** Conditionnements sous gaz comprimé pour aérosol selon la revendication 3, caractérisés en ce que la proportion de monooléate de polyoxyéthylène-20-glycéryle selon la formule III :

$HC-(CH_2)_7-CH_3$

||

$CH(CH_2)_7-C-O-CH_2-CH-OH$

|| |

O $CH_2(OCH_2CH_2)_m-OH$

se situe, rapporté au poids total de mélange, entre 0,75 et 1,5 % en poids.

**13.** Conditionnements sous gaz comprimé pour aérosol selon les revendications 1 à 12,
caractérisés en ce que
l'on utilise comme gaz propulseur TG 227 et/ou TG 134a.

**14.** Procédé de fabrication de conditionnements sous gaz comprimé pour aérosol selon les revendications 1 à 12,
caractérisé en ce qu'
on mélange l'agent propulseur TG 227 et/ou TG 134a tout en refroidissant ou sous pression avec le trioléate de polyoxyéthylène-25-glyécéryle, et/ou le monooléate de polyoxyéthylène-30-glycéryle et/ou le monooléate de polyoxyéthylène-20-glycéryle ainsi que le cas échéant avec des co-solvants, ensuite avec des principes actifs et le cas échéant des substances auxiliaires, on homogénéise la suspension ainsi obtenue et on complète avec l'agent propulseur TG 2257 et/ou TG 134a et on répartit en récipients résistant à la pression.